# EUROPEAN PATENT SPECIFICATION

(11) **EP 2 381 786 B1**
(45) Date of publication and mention of the grant of the patent: **24.12.2014**
(21) Application number: 09839083.4
(22) Date of filing: 08.12.2009
(51) Int. Cl.: A01N 57/00, A61K 31/34, A61P 31/18, C07D 493/04

(54) **DIMETHYLSULFOXIDE SOLVATE OF DARUNAVIR**
DIMETHYLSULFOXID-SOLVAT VON DARUNAVIR
SOLVATE DE DIMETHOXYSULFOXYDE DU DARUNAVIR

(30) Priority: 29.01.2009 US 148055 P; 16.09.2009 US 242818 P
(43) Date of publication of application: 02.11.2011
(60) Divisional application: 14187453.7
(73) Proprietor: Mapi Pharma Limited, 74140 Ness Ziona (IL)
(72) Inventor: MAROM, Ehud, 44308 Kfar Saba (IL)
(74) Representative: Becker Kurig Straus
(86) International application number: PCT/IL2009/001158
(87) International publication number: WO 2010/086844

(56) References cited:
- US-A1- 2005 250 845
- US-A1- 2006 135 562
- US-B2- 6 613 743
- GHOSH A K ET AL: "STEREOSELECTIVE PHOTOCHEMICAL 1,3-DIOXOLANE ADDITION TO 5-ALKOXYMETHYL-2(5H)-FURANONE: SYNTHESIS OF BIS-TETRAHYDROFURANYL LIGAND FOR HIV PROTEASE INHIBITOR UIC-94017 (TMC-114)", JOURNAL OF ORGANIC CHEMISTRY, AMERICAN CHEMICAL SOCIETY, EASTON.; US, vol. 69, no. 23, 21 October 2004 (2004-10-21), pages 7822-7829, XP002465318, ISSN: 0022-3263, DOI: 10.1021/JO049156Y
- GUILLORY J K ED - BRITTAIN H G: "GENERATION OF POLYMORPHS, HYDRATES, SOLVATES, AND AMORPHOUS SOLIDS", POLYMORPHISM IN PHARMACEUTICAL SOLIDS, XX, XX, 1 January 1999 (1999-01-01), pages I-II,183, XP002350313, ISBN: 978-0-8247-0237-9

## Description

### FIELD OF THE INVENTION

The present invention relates to a crystalline dimethylsulfoxide solvate of darunavir, pharmaceutical compositions comprising it, and use thereof in treating retroviral infections.

### BACKGROUND OF THE INVENTION

Darunavir is a second-generation protease inhibitor used for treating human immunodeficiency virus (HIV) infection. Co-administration of darunavir with the antiretroviral drug ritonavir was approved by the FDA in 2006 for the treatment of HIV patients who have already been administered with other antiretroviral drugs.

Darunavir is chemically named [(1S,2R)-3-[[(4-aminophenyl)sulfonyl](2-methylpropyl)amino]-2-hydroxy-1-(phenylmethyl)propyl]-carbamic acid (3R,3aS,6aR)-hexahydrofuro[2,3-b]furan-3-yl ester, and is represented by the following chemical structure:

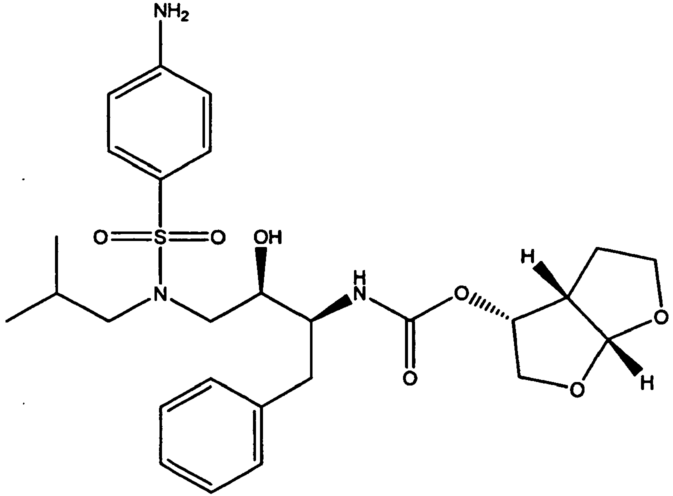

Darunavir and processes for its preparation are disclosed in EP 715618, WO 99/67417, US 5,968,942, US 6,248,775 and in Bioorganic and Chemistry Letters, 8, 687-690, 1998.

Several pseudopolymorphic forms of darunavir are described in US 2005/0250845 including the ethanolate, hydrate, methanolate, acetonate, dichloromethanate, ethylacetate solvate, 1-methoxy-2-propanolate, anisolate, tetrahydrofuranate, isopropanolate and mesylate solvates of darunavir.

Darunavir ethanolate is marketed in the United States under the trade name PREZISTA® by Tibotec. PREZISTA® is available as an orange, oval-shaped, film coated tablet for oral administration. Darunavir monoethanolate solvate is a white to off-white powder with solubility of approximately 0.15 mg/mL in water at 20°C.

A new form of a compound may possess physical properties that differ from, and are advantageous over, those of other crystalline or amorphous forms. These include, packing properties such as molar volume, density and hygroscopicity; thermodynamic properties such as melting temperature, vapor pressure and solubility; kinetic properties such as dissolution rate and stability under various storage conditions; surface properties such as surface area, wettability, interfacial tension and shape; mechanical properties such as hardness, tensile strength, compactibility, handling, flow and blend; and filtration properties. Variations in any one of these properties affect the chemical and pharmaceutical processing of a compound as well as its bioavailability and may often render the new form advantageous for medical use.

There still remains an unmet need for additional solid state forms of darunavir having good physiochemical properties, desirable bioavailability, and advantageous pharmaceutical parameters.

### SUMMARY OF THE INVENTION

The present invention provides a crystalline dimethylsulfoxide solvate of darunavir, pharmaceutical compositions comprising said compound, a method for its preparation and use thereof in treating retroviral infections and, in particular, HIV infection.

The present invention is based in part on the unexpected finding that the new solvate form disclosed herein possess advantageous physicochemical properties which render its processing as medicaments beneficial. The form of the present invention has good bioavailability as well as desirable stability characteristics enabling its incorporation into a variety of different formulations particularly suitable for pharmaceutical utility.

The present disclosure provides a crystalline tetrahydrofuran solvate of darunavir having an X-ray powder diffraction pattern with diffraction peaks at 2-theta values of about 22.8±0.1 and 16.4±0.1.

Further, the present disclosure provides a crystalline tetrahydrofuran solvate of darunavir having an X-ray powder diffraction pattern with diffraction peaks at 2-theta values of about 22.8±0.1, 16.4±0.1, 22.4±0.1 and 20.9±0.1.

The present disclosure provides furthermore a crystalline tetrahydrofuran solvate of darunavir having at least 3 X-ray diffraction peaks selected from about 6.9±0.1, 11.0±0.1, 13.6±0.1, 16.1±0.1, 16.4±0.1, 17.1±0.1, 18.4±0.1, 20.2±0.1, 20.9±0.1, 22.4±0.1, 22.8±0.1 and 23.2±0.1 degrees 2-theta.

The present disclosure further provides a crystalline tetrahydrofuran solvate of darunavir having an X-ray powder diffraction pattern with diffraction peaks at 2-theta values of about 6.9±0.1, 11.0±0.1, 13.6±0.1, 16.1±0.1, 16.4±0.1, 17.1±0.1, 18.4±0.1, 20.2±0.1, 20.9±0.1, 22.4±0.1, 22.8±0.1 and 23.2±0.1.

The present invention provides a crystalline dimethylsulfoxide solvate of darunavir having an X-ray powder diffraction pattern with diffraction peaks at 2-theta values of about 20.6±0.1 and 21.2±0.1.

In some embodiments, the present invention provides a crystalline dimethylsulfoxide solvate of darunavir having an X-ray powder diffraction pattern with diffraction peaks at 2-theta values of about 20.6±0.1, 21.2±0.1, 16.6±0.1 and 23.0±0.1.

In other embodiments, the present invention provides a crystalline dimethylsulfoxide solvate of darunavir having at least 3 X-ray diffraction peaks selected from about 7.1±0.1, 9.3±0.1, 10.6±0.1, 11.4±0.1, 13.9±0.1, 16.6±0.1, 17.3±0.1, 18.5±0.1, 20.1±0.1, 20.6±0.1, 21.2±0.1, 23.0±0.1, 27.1±0.1 and 28.1±0.1 degrees 2-theta.

In particular embodiments, the present invention provides a crystalline dimethylsulfoxide solvate of darunavir having an X-ray powder diffraction pattern with diffraction peaks at 2-theta values of about 7.1±0.1, 9.3±0.1, 10.6±0.1, 11.4±0.1, 13.9±0.1, 16.6±0.1, 17.3±0.1, 18.5±0.1, 20.1±0.1, 20.6±0.1, 21.2±0.1, 23.0±0.1, 27.1±0.1 and 28.1±0.1.

The present disclosure also provides an amorphous form of darunavir having an IR spectrum with characteristic peaks at about 1454 and 1369 cm⁻¹.

The amorphous form of darunavir has an IR spectrum with characteristic peaks at about 1454, 1369, 771 and 553 cm⁻¹.

In specific embodiments, the present invention provides a pharmaceutical composition comprising as an active ingredient a crystalline dimethylsulfoxide solvate of darunavir of the present invention, and a pharmaceutically acceptable carrier.

In a particular embodiment, the pharmaceutical composition is in the form of a tablet.

In various embodiments, the present invention provides a pharmaceutical composition comprising as an active ingredient a crystalline dimethylsulfoxide solvate of darunavir of the present invention, and a pharmaceutically acceptable carrier for use in treating retroviral infections.

In particular embodiments, the retroviral infection is a human immunodeficiency virus (HIV) infection.

In other embodiments, the pharmaceutical composition of the present invention is co-administered in combination with another antiretroviral drug. An exemplary and nonlimiting embodiment is the co-administration with ritonavir.

In some embodiments, the present disclosure provides a method of inhibiting retrovirus protease activity comprising administering to a subject in need thereof an effective amount of a composition comprising any one of the darunavir forms of the present invention.

In additional embodiments, the present invention provides use of a crystalline dimethylsulfoxide solvate of darunavir of the present invention for the preparation of a medicament for inhibiting retrovirus protease activity.

In particular embodiments, the use disclosed herein is designated for inhibiting HIV protease activity.

In specific embodiments, the subject is a mammal, preferably a human.

Further embodiments and the full scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### BRIEF DESCRIPTION OF THE FIGURES

**Figure 1** is a characteristic X-ray diffraction pattern of the crystalline tetrahydrofuran solvate of darunavir.
**Figure 2** is a characteristic X-ray diffraction pattern of the crystalline tetrahydrofuran solvate of darunavir in comparison to darunavir ethanolate API.
**Figure 3** is a characteristic Thermogravimetric analysis (TGA) of the crystalline tetrahydrofuran solvate of darunavir.
**Figure 4** is a characteristic IR spectrum of the crystalline tetrahydrofuran solvate of darunavir **(4A)** in comparison to darunavir ethanolate API **(4B).**
**Figure 5** is a characteristic Raman spectrum of the crystalline tetrahydrofuran solvate of darunavir.
**Figure 6** is a polarized light micrograph of the crystalline tetrahydrofuran solvate of darunavir.
**Figure 7** is a dynamic vapor sorption (DVS) isotherm plot of the crystalline tetrahydrofuran solvate of darunavir. Sorption is represented by diamonds and desorption is represented by squares.
**Figure 8** are HPLC chromatograms of the crystalline dimethylsulfoxide solvate of darunavir **(panel** A), the crystalline tetrahydrofuran solvate of darunavir **(panel B),** the amorphous darunavir of the present invention **(panel C),** and darunavir ethanolate API **(panel D)** slurry in pH 1.2 buffer. **Panel E** is a chromatogram of darunavir ethanolate API standard solution (STD).
**Figure 9** is a characteristic X-ray diffraction pattern of the crystalline dimethylsulfoxide solvate of darunavir.
**Figure 10** are characteristic X-ray diffraction patterns of the crystalline dimethylsulfoxide solvate of darunavir **(panels B-D)** in comparison to darunavir ethanolate API **(panel F)** and the amorphous darunavir form **(panels A and E).**
**Figure 11** is a characteristic Thermogravimetric analysis (TGA) of the crystalline dimethylsulfoxide solvate of darunavir.
**Figure 12** is a characteristic IR spectrum of the crystalline dimethylsulfoxide solvate of darunavir.
**Figure 13** is a characteristic Raman spectrum of the crystalline dimethylsulfoxide solvate of darunavir.
**Figure 14** is a dynamic vapor sorption (DVS) isotherm plot of the crystalline dimethylsulfoxide solvate of darunavir. Sorption is represented by diamonds and desorption is represented by squares.
**Figure 15** is a characteristic IR spectrum of the amorphous darunavir of the present invention.
**Figure 16** is a characteristic Raman spectrum of the amorphous darunavir of the present invention.
**Figure 17** is a dynamic vapor sorption (DVS) isotherm plot of the amorphous darunavir of the present invention. Sorption is represented by diamonds and desorption is represented by squares.
**Figure 18** is a dynamic vapor sorption (DVS) isotherm plot of darunavir ethanolate API. Sorption is represented by diamonds and desorption is represented by squares.

### DETAILED DESCRIPTION OF THE INVENTION

The present invention is directed to a crystalline dimethylsulfoxide solvate of [(1S,2R)-3-[[(4-aminophenyl)sulfonyl](2-methylpropyl)amino]-2-hydroxy-1-(phenylmethyl)propyl]-carbamic acid (3R,3aS,6aR)-hexahydrofuro[2,3-b]furan-3-yl ester having structural formula:

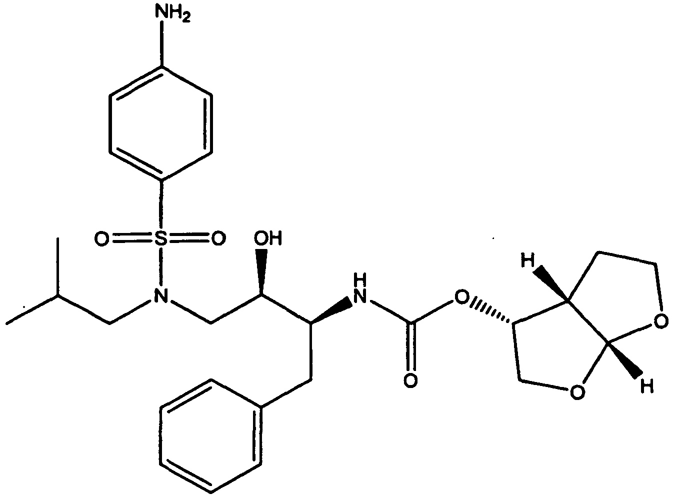

The present invention is further directed to pharmaceutical compositions comprising the said solvate of the present invention and a pharmaceutically acceptable carrier and their use in treating retroviral infections.

Polymorphs are two or more solid state phases of the same chemical compound that possess different arrangement and/or conformation of the molecules. Pseudopolymorphs are polymorphs which incorporate one or more solvents into the structure. Different polymorphs and pseudopolymorphs of an active pharmaceutical compound can exhibit different physical and chemical properties such as color, stability, processability, dissolution and even bioavailability.

An important physical property of a compound used as an active ingredient of a medicament is the stability at ambient conditions, especially to moisture, and under storage conditions. The identification and characterization of various polymorphs and pseudopolymorphs of a pharmaceutically active compound is therefore of great significance in obtaining medicaments with desired properties including a specific dissolution rate, milling property, bulk density, thermal stability or shelf-life. The darunavir forms of the present invention possess improved characteristics of hygroscopicity, bulk density and solubility in aqueous media. Furthermore, the solvate of darunavir of the present invention has improved chemical and solid state stability. Hence, this form may be more stable when stored over prolonged periods of time.

The present disclosure relates to crystalline tetrahydrofuran solvates of darunavir having any stoichiometry from 0.5 tetrahydrofuran to 5.0 molecules of tetrahydrofuran per molecule of darunavir. Exemplary stoichiometries are hemisolvates, monosolvates, disolvates or trisolvates.

Provided herein is a crystalline tetrahydrofuran solvate of darunavir which is characterized by a unique X-ray diffraction pattern having characteristic peaks expressed in degrees 2-theta at about 22.8±0.1 and 16.4±0.1. Preferably, the X-ray diffraction pattern has additional characteristic peaks expressed in degrees 2- theta at about 22.4±0.1 and 20.9±0.1. More preferably, the X-ray diffraction pattern has additional characteristic peaks expressed in degrees 2- theta at about 11.0±0.1, 17.1±0.1 and 20.2±0.1. Most preferably, the X-ray diffraction pattern has characteristic peaks expressed in degrees 2-theta at about 6.9±0.1, 11.0±0.1, 13.6±0.1, 16.1±0.1, 16.4±0.1, 17.1±0.1, 18.4±0.1, 20.2±0.1, 20.9±0.1, 22.4±0.1, 22.8±0.1 and 23.2±0.1.

The crystalline form of darunavir tetrahydrofuran solvate can be further characterized by its melting point and by using various techniques including infrared absorption, Raman spectrometry, solid state NMR, and thermal analysis (e.g. thermogravimetric analysis (TGA) and differential scanning calorimetry (DSC)).

Specifically, the crystalline tetrahydrofuran solvate of darunavir is characterized by TGA as having an approximately 9-11% weight loss at a temperature range of room temperature (RT) to 200°C substantially attributed to solvate release. The form is further characterized by Infrared spectroscopy to have characteristic peaks and their relative intensities¹ at the following wavenumbers: 3437m, 3348s, 3253m, 3062vw, 3030vw, 2961m, 2901w, 2872w, 1704vs, 1646w, 1596vs, 1548m, 1503m, 1455w, 1368w, 1342m, 1317s, 1263s, 1244m, 1227w, 1185w, 1153vs, 1090m, 1044m, 1021m, 988m, 944m, 910w, 885vw, 862vw, 839w, 767m, 741m, 698w, 673m, 632w, 581m, 554s, and 502vw cm⁻¹. The present invention relates to crystalline dimethylsulfoxide solvates of darunavir having any stoichiometry from 0.5 dimethylsulfoxide to 5.0 molecules of
¹ vs= very strong, s=strong, m=medium, w=weak, vw=very weak, br=broad. dimethylsulfoxide per molecule of darunavir. Particular stoichiometries are hemisolvates, monosolvates, disolvates or trisolvates.

Provided herein is a crystalline dimethylsulfoxide solvate of darunavir which is characterized by a unique X-ray diffraction pattern having characteristic peaks expressed in degrees 2- theta at about 20.6±0.1 and 21.2±0.1. Preferably, the X-ray diffraction pattern has additional characteristic peaks expressed in degrees 2- theta at about 16.6±0.1 and 23.0±0.1. More preferably, the X-ray diffraction pattern has additional characteristic peaks expressed in degrees 2- theta at about 18.5±0.1 and 17.3±0.1. Most preferably, the X-ray diffraction pattern has characteristic peaks expressed in degrees 2- theta at about 7.1±0.1, 9.3±0.1, 10.6±0.1, 11.4±0.1, 13.9±0.1, 16.6±0.1, 17.3±0.1, 18.5±0.1, 20.1±0.1, 20.6±0.1, 21.2±0.1, 23.0±0.1, 27.1±0.1 and 28.1±0.1.

Additionally, the crystalline form of darunavir dimethylsulfoxide solvate is characterized by an about 10-12% weight loss at a temperature range of RT to 230°C substantially attributed to solvate release. The form is further characterized by Infrared spectroscopy with characteristic peaks and their relative intensities² at 3407br, 3342s, 3250m, 3062vw, 3026vw, 2962m, 2901w, 2872w, 1704vs, 1646w, 1596vs, 1546m, 1500m, 1467w, 1454w, 1372w, 1340m, 1311s, 1263s, 1244m, 1227w, 1183w, 1155vs, 1091m, 1043m, 1023m, 988m, 947m, 891w, 862vw, 842w, 769m, 744m, 700w, 671m, 554s, and 502vw cm⁻¹.

The present disclosure further relates to amorphous darunavir characterized by an X-ray diffraction pattern having a single broad peak expressed between 10 and 25 [2θ°]. The amorphous darunavir of the present invention is further characterized by IR peaks and their relative intensities² at the following wavenumbers: 3466br, 3386s, 3250m, 3066vw, 3026vw, 2960m, 2901w, 2872w, 1706vs, 1633m, 1597vs, 1537m, 1503m, 1454w, 1369w, 1315s, 1260m, 1149vs, 1091s, 1041m, 1017m, 937w, 885vw, 833w, 771m, 702m, 673m, 632w, and 553s cm⁻¹.

The solvate of darunavir of the present invention as well as the herein disclosed THF solvate and the amorphous form can be prepared by a variety of methods, including for example, crystallization/precipitation or recrystallization from a suitable solvent, sublimation,
² vs= very strong, s=strong, m=medium, w=weak, vw=very weak, br=broad. growth from a melt, solid state transformation from another phase, crystallization/precipitation from a supercritical fluid, and jet spraying. Techniques for crystallization or recrystallization of crystalline forms from a solvent mixture include, for example, evaporation of the solvent, decreasing the temperature of the solvent mixture, crystal seeding a supersaturated solvent mixture of the molecule and/or salt, freeze drying the solvent mixture, and addition of antisolvents (countersolvents) to the solvent mixture. The term "antisolvent" as used herein refers to a solvent in which the compound has low solubility.

Suitable solvents and anti-solvents for preparing crystals include polar and nonpolar solvents. The choice of solvent or solvents is typically dependent upon one or more factors, including solubility of the compound in such solvent, crystallization technique, and vapor pressure of the solvent. Combinations of solvents may be employed; for example, the compound may be solubilized into a first solvent followed by the addition of an antisolvent to decrease the solubility of the compound in the solution and to induce crystallization. Suitable solvents include, but are not limited to, polar aprotic solvents and polar protic solvents, and mixtures thereof. Particular examples of suitable polar aprotic solvents include, but are not limited to, acetonitrile, tetrahydrofuran (THF), dichloromethane, acetone, dimethylformamide, and dimethylsulfoxide.

Seed crystals may be added to any crystallization mixture to promote crystallization as is known in the art. Alternatively, crystalline forms may be obtained by distillation or solvent addition techniques such as those known to those skilled in the art. Suitable solvents for this purpose include any of those solvents described herein, including protic polar solvents, such as alcohols (for example, methanol, ethanol, and isopropanol), aprotic polar solvents (including those listed above), and also ketones (for example, acetone, methyl ethyl ketone, and methyl isobutyl ketone).

Exemplary processes used to prepare each of the pseudopolymorphic darunavir forms of the present invention are provided herein.

Methods for "crystallization from solution" include, but are not limited to, a concentration method, a slow cooling method, a reaction method (diffusion method, electrolysis method), a hydrothermal growth method, a fusing agent method, and so forth. The solution can be a supersaturated solution, optionally heated to temperatures bellow the solvent boiling point. The recovery of the solid state forms can be done for example, by filtering the suspension and drying.

In particular, the darunavir form of the present invention can be prepared by the slurry method as is well known in the art. Suspensions of the active ingredient in different solvents or mixture of solvents are prepared and shaken for long intervals (typically 24 hours).

The darunavir form of the present invention can be prepared using slow precipitation from saturated solutions in different solvents or mixture of solvents which are allowed to evaporate at room temperatures. Alternatively the saturated solutions can be heated followed by their cooling to induce precipitation as is known in the art.

Encompassed by the present invention are methods of antisolvent precipitation where an antisolvent is added to the saturated solution of the active ingredient in different solvents or mixture of solvents to induce precipitation.

Within the scope of the present invention are high pressure techniques where the active ingredient is compressed using various forces as is known in the art.

The novel form of darunavir is useful as pharmaceutical for inhibiting retroviral infections. The present invention thus provides pharmaceutical compositions comprising the said solvate disclosed herein and a pharmaceutically acceptable carrier. The solid state of the present invention can be safely administered orally or non-orally (e. g., topical, rectal). The pharmaceutical compositions can be formulated as tablets (including sugar-coated tablets and film-coated tablets), powders, granules, capsules (including soft capsules), orally disintegrating tablets, and sustained-release preparations as is well known in the art.

Pharmacologically acceptable carriers that may be used in the context of the present invention include various organic or inorganic carriers including, but not limited to, excipients, lubricants, binders, disintegrants, water-soluble polymers and basic inorganic salts. The pharmaceutical compositions of the present invention may further include additives such as, but not limited to, preservatives, antioxidants, coloring agents, sweetening agents, souring agents, bubbling agents and flavorings.

Suitable excipients include e.g. lactose, sucrose, D-mannitol, starch, cornstarch, crystalline cellulose, light silicic anhydride and titanium oxide. Suitable lubricants include e.g. magnesium stearate, sucrose fatty acid esters, polyethylene glycol, talc and stearic acid. Suitable binders include e.g. hydroxypropyl cellulose, hydroxypropylmethyl cellulose, crystalline cellulose, a-starch, polyvinylpyrrolidone, gum arabic powder, gelatin, pullulan and low-substitutional hydroxypropyl cellulose. Suitable disintegrants include e.g. crosslinked povidone (any crosslinked 1-ethenyl-2-pyrrolidinone homopolymer including polyvinylpyrrolidone (PVPP) and 1-vinyl-2-pyrrolidinone homopolymer), crosslinked carmellose sodium, carmellose calcium, carboxymethyl starch sodium, low-substituted hydroxypropyl cellulose, cornstarch and the like. Suitable water-soluble polymers include e.g. cellulose derivatives such as hydroxypropyl cellulose, polyvinylpyrrolidone, hydroxypropylmethyl cellulose, methyl cellulose and carboxymethyl cellulose sodium, sodium polyacrylate, polyvinyl alcohol, sodium alginate, guar gum and the like. Suitable basic inorganic salts include e.g. basic inorganic salts of sodium, potassium, magnesium and/or calcium. Particular embodiments include the basic inorganic salts of magnesium and/or calcium. Basic inorganic salts of sodium include, for example, sodium carbonate, sodium hydrogen carbonate, disodiumhydrogenphosphate, etc. Basic inorganic salts of potassium include, for example, potassium carbonate, potassium hydrogen carbonate, etc. Basic inorganic salts of magnesium include, for example, heavy magnesium carbonate, magnesium carbonate, magnesium oxide, magnesium hydroxide, magnesium metasilicate aluminate, magnesium silicate, magnesium aluminate, synthetic hydrotalcite, aluminahydroxidemagnesium and the like. Basic inorganic salts of calcium include, for example, precipitated calcium carbonate, calcium hydroxide, etc.

Suitable preservatives include e.g. sodium benzoate, benzoic acid, and sorbic acid. Suitable antioxidants include e.g. sulfites, ascorbic acid and a-tocopherol. Suitable coloring agents include e.g. food colors such as Food Color Yellow No. 5, Food Color Red No. 2 and Food Color Blue No. 2 and the like. Suitable sweetening agents include e.g. saccharin sodium, dipotassium glycyrrhetinate, aspartame, stevia and thaumatin. Suitable souring agents include e.g. citric acid (citric anhydride), tartaric acid and malic acid. Suitable bubbling agents include e.g. sodium bicarbonate. Suitable flavorings include synthetic substances or naturally occurring substances, including e.g. lemon, lime, orange, menthol and strawberry.

The solid form of the present invention is particularly suitable for oral administration in the form of tablets, capsules, pills, dragées, powders, granules and the like. A tablet may be made by compression or molding, optionally with one or more excipients as is known in the art. Specifically, molded tablets may be made by molding in a suitable machine a mixture of the powdered active ingredient moistened with an inert liquid diluent.

The tablets and other solid dosage forms of the pharmaceutical compositions described herein may optionally be scored or prepared with coatings and shells, such as enteric coatings and other coatings well known in the art. They may also be formulated so as to provide slow or controlled release of the active ingredient therein using, for example, hydroxypropylmethyl cellulose in varying proportions to provide the desired release profile, other polymer matrices and the like. The active ingredient can also be in micro-encapsulated form, if appropriate, with one or more of the above-described excipients.

The present invention provides a method for use in inhibiting retrovirus protease activity comprising administering to a subject in need thereof an effective amount of a composition comprising any one of the darunavir forms of the present invention.

"A therapeutically effective amount" as used herein refers to an amount of an agent which is effective, upon single or multiple dose administration to the subject in providing a therapeutic benefit to the subject. In one embodiment, the therapeutic benefit is inhibiting retrovirus protease activity, or in prolonging the survivability of a subject with such a viral infection beyond that expected in the absence of such treatment. In additional embodiments, the darunavir forms of the present invention are used for the preparation of a medicament for treating diseases caused by retroviruses such as HIV infections, e.g. Acquired Immune Deficiency Syndrome (AIDS) and AIDS-Related Complex (ARC).

The present invention further provides the administration of the said solvate of darunavir in combination therapy with 1 to 3 other active ingredients. Such "other active ingredients", according to the principles of the present invention include, but are not limited to, other antiretroviral drugs (e.g. Etravirine, Raltegravir, Rifabutin). In specific embodiments, the present invention provides the co-administration of darunavir with ritonavir.

It is further contemplated that the combination therapy will include the two or more active ingredients within a single pharmaceutical composition as well as the two or more active ingredients in two separate pharmaceutical compositions administered to the same subject simultaneously or at a time interval determined by a skilled artisan.

The principles of the present invention are demonstrated by means of the following non-limitative examples.

### EXAMPLES

### Reference Example 1: Preparation of the crystalline tetrahydrofuran solvate of darunavir

Darunavir tetrahydrofuran solvate was prepared by dissolving about 1g of Darunavir ethanolate in 5ml of a tetrahydrofuran solvent. The solvent was then allowed to evaporate at room temperature (approximately 25°C) until crystals were formed.

Alternatively, the darunavir tetrahydrofuran solvate was prepared by dissolving Darunavir ethanolate in a tetrahydrofuran solvent, followed by the addition of the antisolvent isopropanol (IPA) to induce precipitation of the crystals.

Alternatively, the darunavir tetrahydrofuran solvate was prepared by dissolving Darunavir ethanolate in tetrahydrofuran (THF): isopropyl acetate (iPrOAc) at a ratio of 1: 2 or tetrahydrofuran (THF): methyl tert-butyl ether (MTBE) at a ratio of 1: 2, heating the mixture to 60°C, followed by cooling using an ice-bath to induce crystallization.

### Reference Example 2: Characterization of the crystalline tetrahydrofuran solvate of darunavir

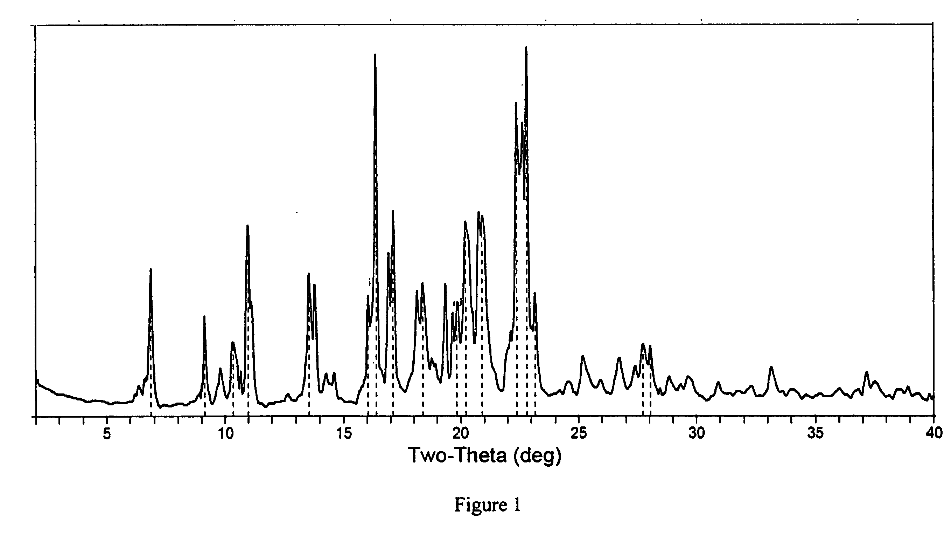

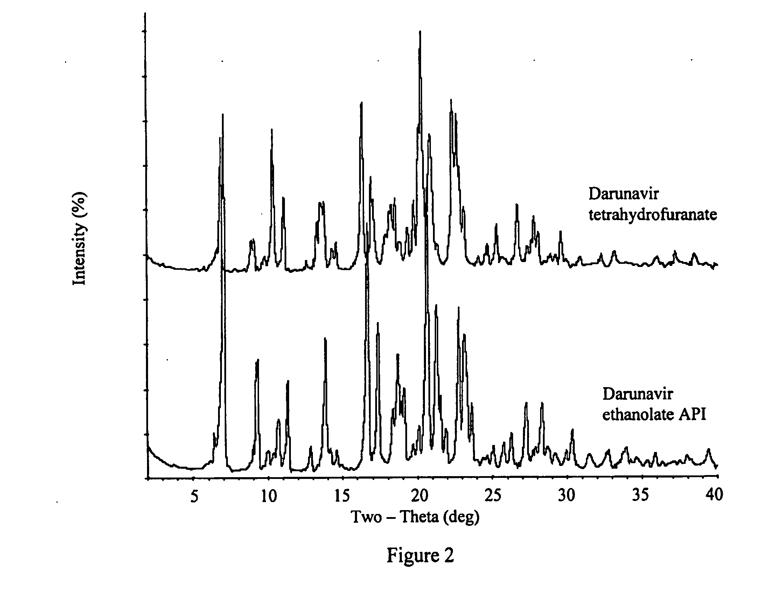

This polymorphic form showed an endothermic peak in Differential Scanning Calorimetry (DSC; Mettler Toledo DSC 1; 10°C/min) at ~95°C. X-ray powder diffraction (XRPD; Rigaku D/MAX 2200, CuKα, 40kV, 40mA, DivSlit 1deg, DivH.L.Slit 10mm, SctSlit 1deg, RecSlit 0.3mm, 10deg/min) shows unique characteristic peaks (Figure 1; Table 1). The X-ray diffraction pattern of the tetrahydrofuran solvate of darunavir has a unique fingerprint which differs from the X-ray diffraction pattern of darunavir ethanolate API (Figure 2). The XRPD and DSC spectra remained unchanged even after storage at 25°C for 2 weeks, thus indicating crystal stability.

**Table 1: X-ray diffraction peaks of darunavir tetrahydrofuran solvate**

| 2-theta | d-spacing [Å] | Width at half height | Relative intensity* (%) |
|---|---|---|---|
| 6.898 | 12.8032 | 0.213 | 39.7 |
| 9.142 | 9.6656 | 0.153 | 26.8 |
| 10.340 | 8.5484 | 0.338 | 19.9 |
| 10.980 | 8.0518 | 0.253 | 51.8 |
| 13.579 | 5.5155 | 0.223 | 38.2 |
| 16.060 | 5.5143 | 0.377 | 32.3 |
| 16.398 | 5.4013 | 0.232 | 98.9 |
| 17.123 | 5.1744 | 0.174 | 54.1 |
| 18.380 | 4.8231 | 0.440 | 35.8 |
| 19.824 | 4.4749 | 0.512 | 26.8 |
| 20.221 | 4.3879 | 0.337 | 52.8 |
| 20.920 | 4.2428 | 0.411 | 54.2 |
| 22.381 | 3.9691 | 0.563 | 86.4 |
| 22.801 | 3.8970 | 0.325 | 100.0 |
| 23.160 | 3.8374 | 0.427 | 33.0 |
| 27.740 | 3.2133 | 0.528 | 19.2 |
| 28.041 | 3.1795 | 0.528 | 18.9 |

| | | | |
|---|---|---|---|
| * Relative intensities may vary among samples. | | | |

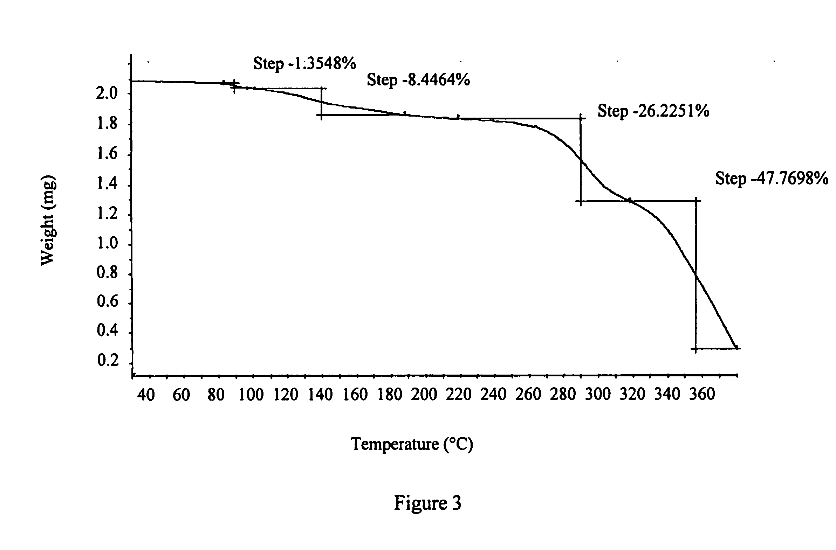

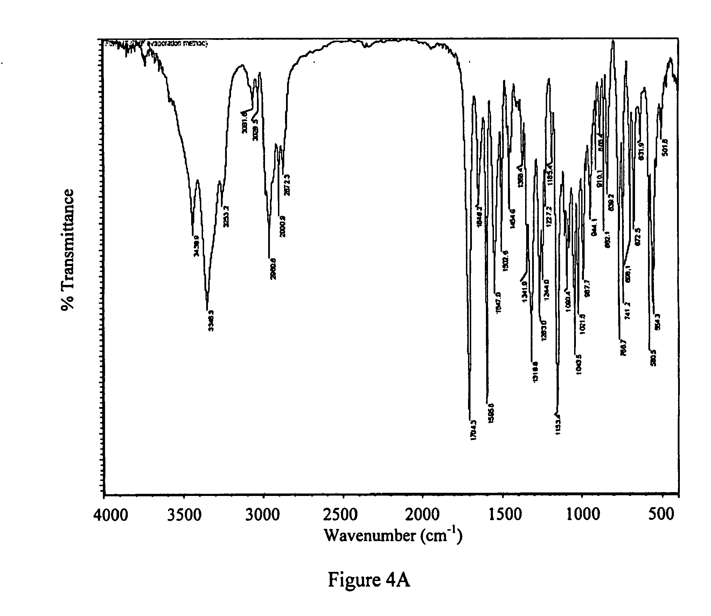

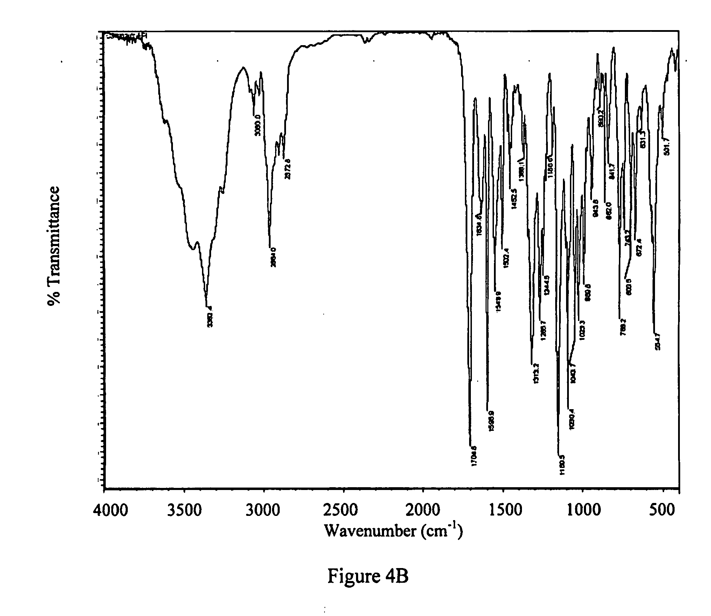

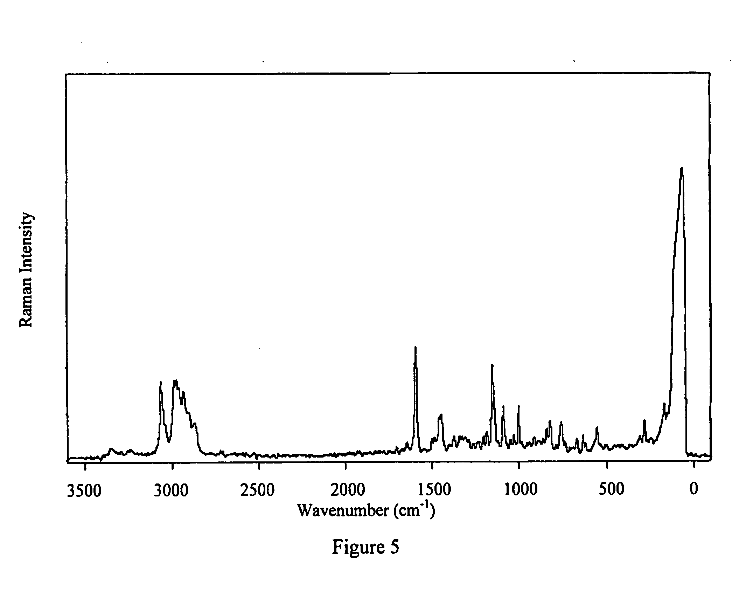

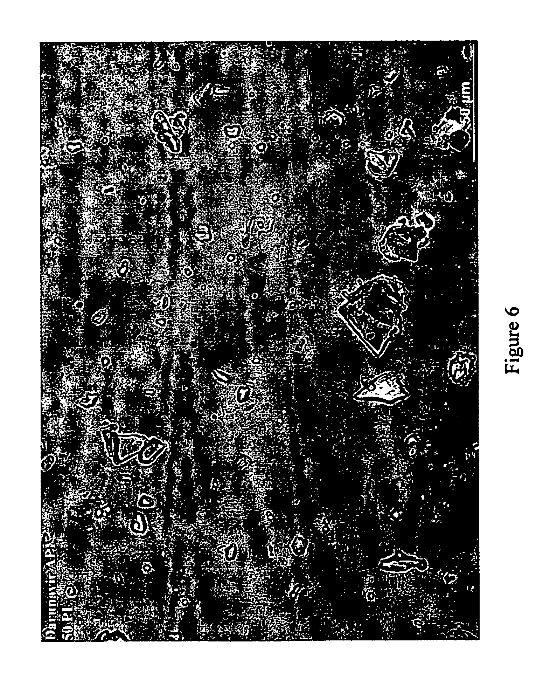

Thermogravimetric analysis (TGA; Mettler Toledo TGA/DSC 1100, 10°C/min) showed a weight loss of approximately 1.3% at temperatures of 60-100°C and another weight loss of approximately 8.5% at temperatures of 100-190°C (Figure 3). Infrared (IR) spectroscopy revealed significant differences between the tetrahydrofuran solvate form and the known ethanolate form, particularly at the alcohol region (3100-3400 cm⁻¹; Figure 4A and 4B, respectively). The Raman spectrum is shown in Figure 5. The characteristic Raman peaks of the darunavir tetrahydrofuran solvate appear at about 62, 171, 283, 555, 636, 672, 760, 824, 1004, 1091, 1155, 1376, 1448, 1596, 1647, 2871, 2937, 2974, and 3064 cm⁻¹. Differences in the Raman spectra between the tetrahydrofuran solvate form and the known ethanolate form of darunavir appear at least at the following wavenumbers (cm⁻¹): 2871, 1647, 1376 and 1155. Polarized light microscopy of the crystals revealed small birefringent plates (Nikon LV100POL equipped with 5 megapixel CCD, Physical lens 50×; Figure 6). The bulk density of the darunavir tetrahydrofuran solvate of the present invention is 0.431±0.007 g/ml.

About 10mg of the tetrahydrofuran solvate form were used to test the hygroscopicity (DVS) from 0% to 90% according to the details on Table 2.

**Table 2: Parameters for hygroscopicity measurements (dynamic vapor sorption; DVS)**

| Stage number | Stage type | dm/dt (%/min) | Start PP (%) | Stop PP (%) | Temp (°C) |
|---|---|---|---|---|---|
| 1 | dm/dt | 0.002 | 10 | 0.0 | 25.0 |
| 2 | dm/dt | 0.002 | 10.0 | 10.0 | 25.0 |
| 3 | dm/dt | 0.002 | 20.0 | 20.0 | 25.0 |
| 4 | dm/dt | 0.002 | 30.0 | 30.0 | 25.0 |
| 5 | dm/dt | 0.002 | 40.0 | 40.0 | 25.0 |
| 6 | dm/dt | 0.002 | 50.0 | 50.0 | 25.0 |
| 7 | dm/dt | 0.002 | 50.0 | 60.0 | 25.0 |
| 8 | dm/dt | 0.002 | 70.0 | 70.0 | 25.0 |
| 9 | dm/dt | 0.002 | 80.0 | 80.0 | 25.0 |
| 10 | dm/dt | 0.002 | 90.0 | 90.0 | 25.0 |
| 11 | dm/dt | 0.002 | 80.0 | 80.0 | 25.0 |
| 12 | dm/dt | 0.002 | 70.0 | 70.0 | 25.0 |
| 13 | dm/dt | 0.002 | 60.0 | 50.0 | 25.0 |
| 14 | dm/dt | 0.002 | 50.0 | 50.0 | 25.0 |
| 15 | dm/dt | 0.002 | 40.0 | 40.0 | 25.0 |
| 16 | dm/dt | 0.002 | 30.0 | 30.0 | 25.0 |
| 17 | dm/dt | 0.002 | 20.0 | 20.0 | 25.0 |
| 18 | dm/dt | 0.002 | 10.0 | 10.0 | 25.0 |
| 19 | dm/dt | 0.002 | 0.0 | 0.0 | 25.0 |

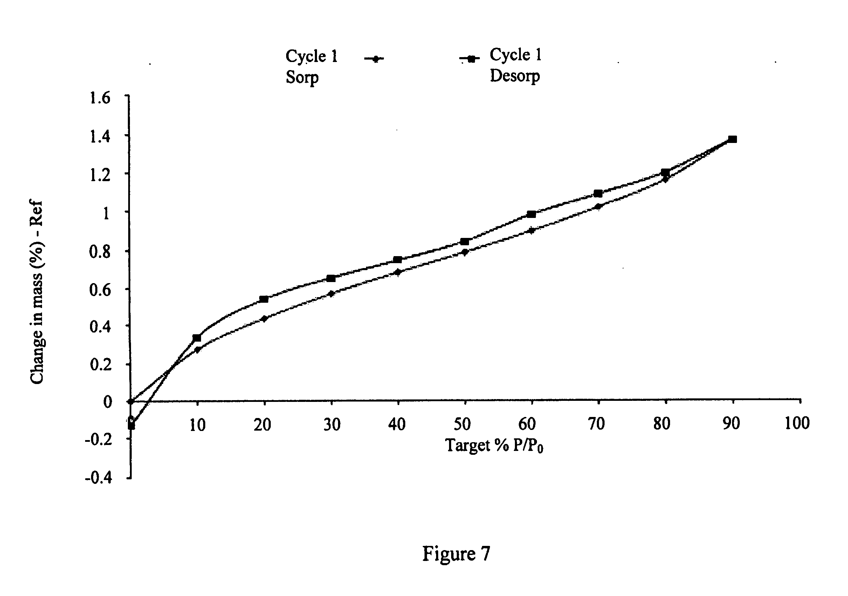

The tetrahydrofuran solvate form was found to be slightly hygroscopic (1.366% weight gain from 0% to 90%; Figure 7).

The tetrahydrofuran solvate of darunavir was further evaluated for its chemical stability. The results are summarized in Table 3. Specifically, about 3 mg of the compound were weighed accurately into a 20 ml clear glass vial and stored under the following conditions: 40°C, 60°C, 40°C/RH 75%, 60°C/RH 75%, and light (25°C), for 2 weeks. A sample stored at -20°C was used as control. A slight increase in Total Related Substances (TRS) was found at 40 °C which was more significant at 60 °C and 60 °C/75%RH. In contrast, no increase was observed when the tetrahydrofuran solvate was stored under exposure to light at 25 °C, both at the end of 1^{st} and 2^{nd} week. Additionally, no change was observed in the physical appearance at the end of the 1^{st} and 2^{nd} week for samples stored at 40°C, 40°C/RH 75% and 25 °C under light. Samples that were stored at 60 °C and 60 °C/75%RH were found stuck to the glass vial.

**Table 3: Solid stability of darunavir tetrahydrofuran solvate at 40°C, 60°C, 40°C/75%RH, 60°C/75%RH and under exposure to light for 7 days and 14 days**

| Condition | Time | Sample number | Weight (mg) | Appearance | TRS% | Remaining% |
|---|---|---|---|---|---|---|
| -20°C | 7d | 1 | 2.446 | No change | 3.54 | - |
| | | 2 | 2.547 | No change | 3.52 | |
| | 14d | 1 | 1.935 | No change | 3.44 | - |
| | | 2 | 3.040 | No change | 3.53 | |
| 40°C | 7d | 1 | 2.469 | No change | 4.11 | 99.83 |
| | | 2 | 2.775 | No change | 4.02 | |
| | 14d | 1 | 2.628 | No change | 4.08 | 99.64 |
| | | 2 | 3.422 | No change | 4.15 | |
| 60°C | 7d | 1 | 2.903 | Stuck | 6.25 | 97.24 |
| | | 2 | 2.222 | Stuck | 5.70 | |
| | 14d | 1 | 3.166 | Stuck | 8.88 | 94.12 |
| | | 2 | 2.512 | Stuck | 9.37 | |
| 40°C/75%RH | 7d | 1 | 2.532 | No change | 2.84 | 100.77 |
| | | 2 | 2.824 | No change | 3.17 | |
| | 14d | 1 | 2.227 | No change | 2.82 | 101.52 |
| | | 2 | 2.681 | No change | 2.65 | |
| 60°C/75%RH | 7d | 1 | 2.479 | Stuck | 5.05 | 97.84 |
| | | 2 | 2.650 | Stuck | 4.17 | |
| | 14d | 1 | 2.869 | Stuck | 4.34 | 98.59 |
| | | 2 | 2.856 | Stuck | 5.19 | |
| light | 7d | 1 | 0.002 | No change | 3.56 | 100.02 |
| | | 2 | 2.859 | No change | 3.64 | |
| | 14d | 1 | 2.345 | No change | 3.63 | 100.78 |
| | | 2 | 3.012 | No change | 3.57 | |

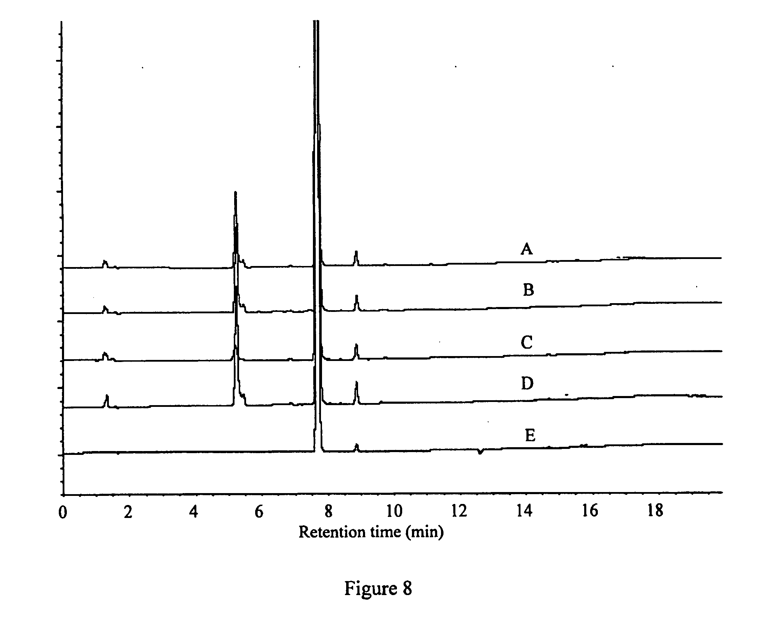

The aqueous solubility of the tetrahydrofuran solvate of darunavir was measured. Specifically, about 10 mg of the compound was accurately weighed into a 4 ml clear glass vial followed by the addition of 2.5 ml buffer (at different pH) to the vial. The vial was then mounted on a Thermomixer and was kept shaking for 24 hours at 25°C. The solution was filtered through 0.45 µm PTFE filter. The pH value and concentration of the filtrate were checked by pH meter and HPLC (Agilent 1200; Column: Zorbax SB C18, 4.6mm ×150 mm ID × 5µm; Profile of mobile phase: t=0 water 70, ACN 30; t=15,20 water 0, ACN 100; Column temperature 30°C; Mobile rate 1.0 mL/min; Detector wavelength 265 nm; The typical retention time of Darunavir is 7.7 min), respectively. The results are summarized in Table 4 and Figure 8 panel B.

**Table 4: Solubility results of darunavir tetrahydrofuran solvate in aqueous buffers**

| Testing media | Solubility (mg/ml) | Appearance | Final pH |
|---|---|---|---|
| water | 0.27 | Many particles | 5.664 |
| pH 1.2 | 1.47* | Many particles | 1.197 |
| pH 4.5 | 0.27 | Many particles | 4.508 |
| pH 6.8 | 0.24 | Many particles | 6.789 |
| pH 7.4 | 0.23 | Many particles | 7.431 |

| | | | |
|---|---|---|---|
| *degraded | | | |

### Example 3: Preparation of the crystalline dimethylsulfoxide solvate of darunavir

Darunavir dimethylsulfoxide solvate of the present invention was prepared by dissolving Darunavir ethanolate in dimethylsulfoxide at 60°C followed by cooling using an ice-bath to induce crystallization.

Alternatively, the darunavir dimethylsulfoxide solvate of the present invention was prepared by dissolving about 1g of Darunavir ethanolate in 2.5ml dimethylsulfoxide at 80°C. Water (10ml) was then added to induce crystallization.

Alternatively, the darunavir dimethylsulfoxide solvate of the present invention was prepared by dissolving Darunavir ethanolate in dimethylsulfoxide followed by the addition of the antisolvent isopropanol (IPA) to induce precipitation of the crystals.

Alternatively, the darunavir dimethylsulfoxide solvate of the present invention was prepared by dissolving Darunavir ethanolate in either one of the following solvent mixtures: dimethylsulfoxide (DMSO):methanol (MeOH) at a ratio of 1:10, dimethylsulfoxide (DMSO):toluene at a ratio of 1:10 or dimethylsulfoxide (DMSO): ethanol (EtOH) at a ratio of 1:10 at 60°C. The mixtures were then allowed to evaporate until crystals were formed.

### Example 4: Characterization of the crystalline dimethylsulfoxide solvate of darunavir

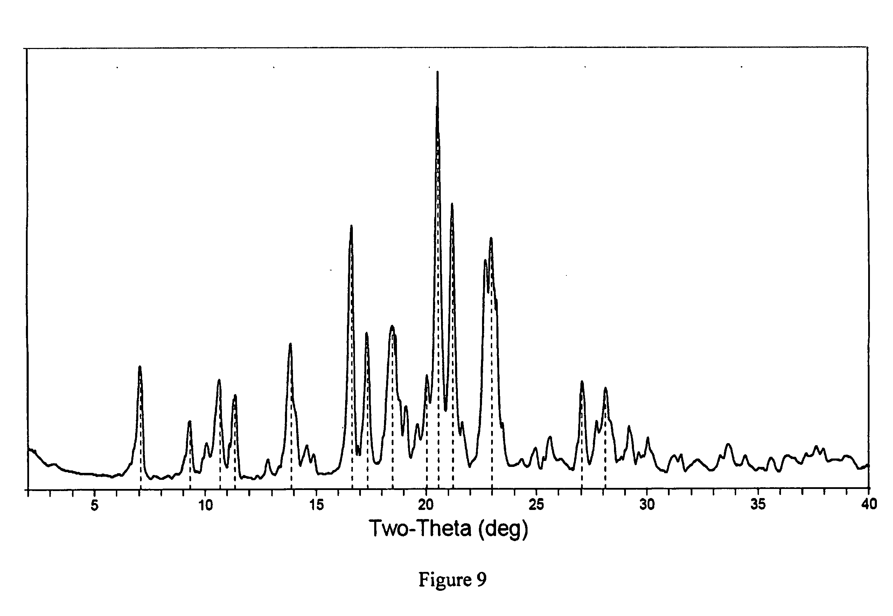

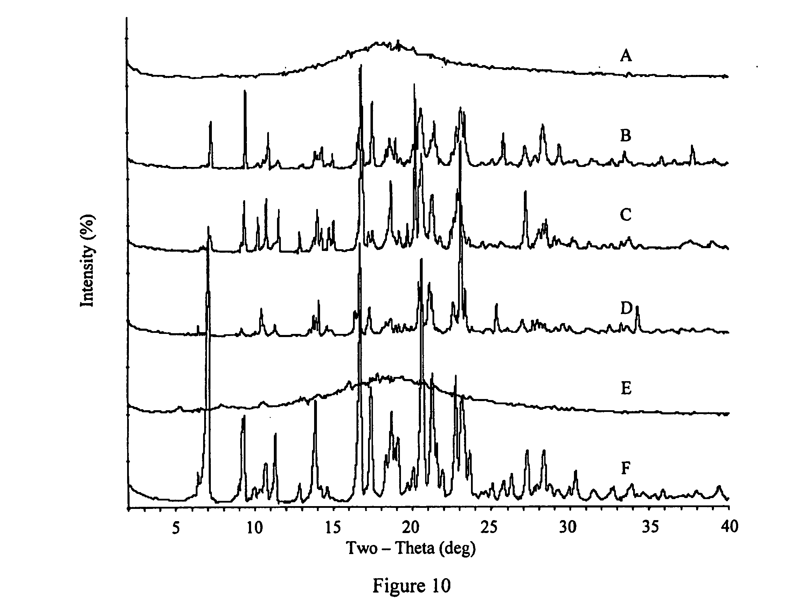

The crystalline dimethylsulfoxide solvate of darunavir showed an endothermic peak at ∼115°C using Differential Scanning Calorimetry. The X-ray powder diffraction of the darunavir dimethylsulfoxide solvate of the present invention is presented in Figure 9 and Table 5. The X-ray diffraction pattern shows a unique fingerprint (Figure 10, panels B, C, and D) which differs from the diffraction pattern of the known ethanolate crystalline form (Figure 10, panel F). The XRPD and DSC spectra remained unchanged even after storage at 25°C for 2 weeks indicating crystal stability.

**Table 5: X-ray diffraction peaks of darunavir dimethylsulfoxide solvate**

| 2-theta | d-spacing [Å] | Width at half height | Relative intensity* (%) |
|---|---|---|---|
| 7.080 | 12.4755 | 0.277 | 29.5 |
| 9.318 | 9.4832 | 0.247 | 16.2 |
| 10.640 | 8.3078 | 0.461 | 26.3 |
| 11.361 | 7.7826 | 0.277 | 22.5 |
| 13.878 | 6.3759 | 0.332 | 34.9 |
| 16.640 | 5.3232 | 0.281 | 53.7 |
| 17.340 | 5.1101 | 0.214 | 37.6 |
| 18.500 | 4.7922 | 0.543 | 39.2 |
| 20.060 | 4.4228 | 0.535 | 27.2 |
| 20.580 | 4.3123 | 0.326 | 100.0 |
| 21.200 | 4.1874 | 0.310 | 68.7 |
| 22.999 | 3.8638 | 0.594 | 60.7 |
| 27.060 | 3.2925 | 0.254 | 25.7 |
| 28.140 | 3.1685 | 0.456 | 24.1 |

| | | | |
|---|---|---|---|
| * Relative intensities may vary among samples. | | | |

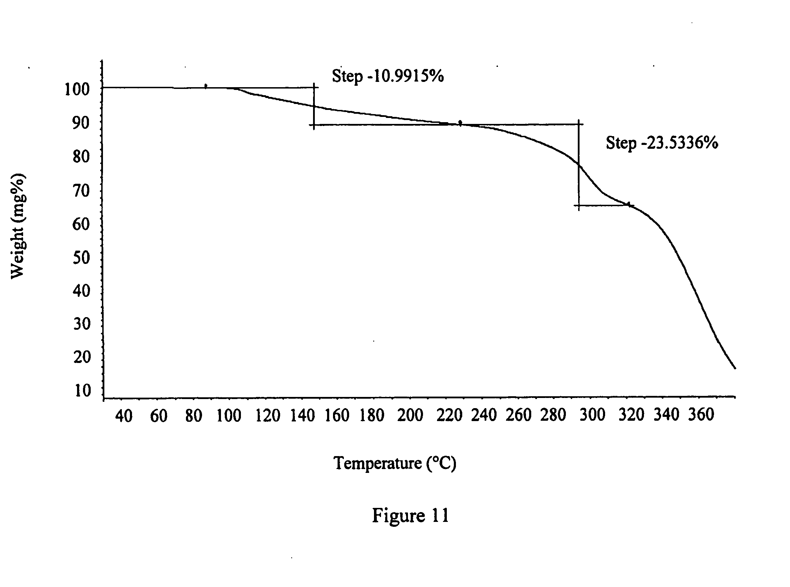

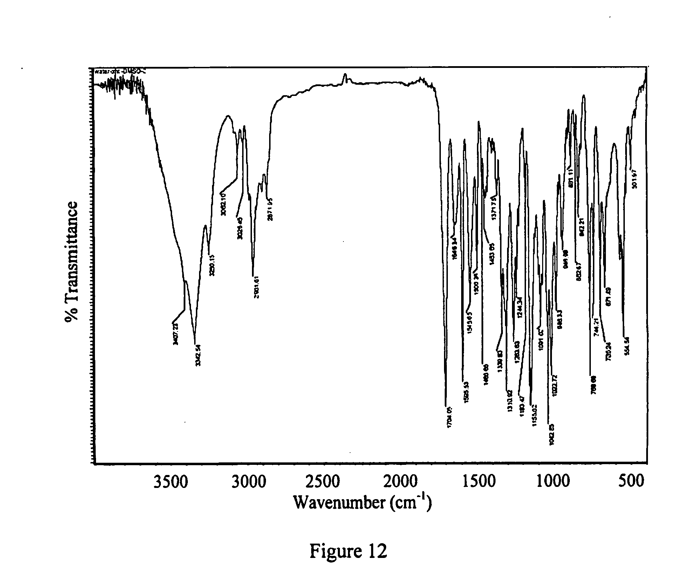

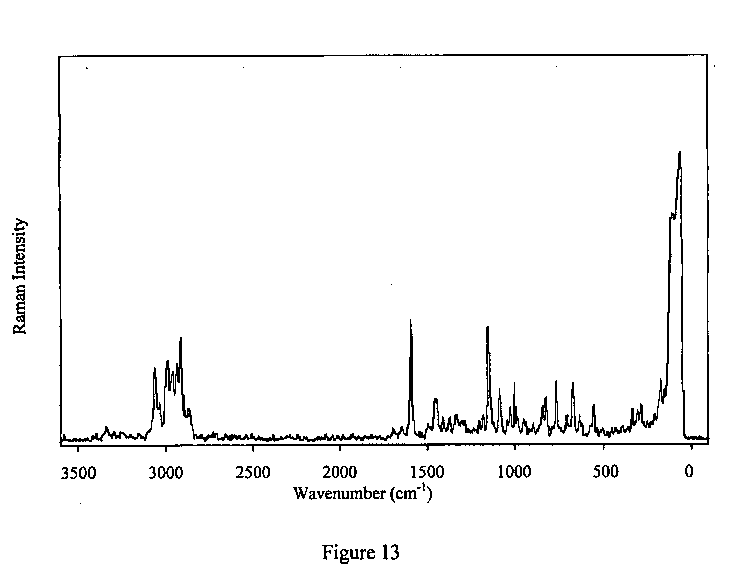

Thermogravimetric analysis of darunavir dimethylsulfoxide solvate showed a weight loss of approximately 11% at a temperature range of RT- 230°C substantially attributed to the release of solvate molecules (Figure 11). Figure 12 shows the Infrared (IR) spectrum of the darunavir dimethylsulfoxide solvate of the present invention which has significant differences from other known forms of darunavir, particularly at the alcohol region (3100-3400 cm⁻¹). The Raman spectrum is shown in Figure 13. The characteristic Raman peaks of the darunavir dimethylsulfoxide solvate of the present invention appear at about 108, 172, 284, 333, 391, 555, 673, 707, 768, 824, 954, 1004, 1031, 1081, 1155, 1184, 1208, 1291, 1341, 1375, 1414, 1459, 1595, 1649, 1700, 2871, 2915, 2937, 2962, 2989, 3064, and 3340 cm⁻¹. Differences in the Raman intensity between the dimethylsulfoxide solvate form of the present invention and the known ethanolate form of darunavir appear at least at the following wavenumbers (cm⁻¹): 3340, 2915, 2871, 1700, 1649, 1414, 1375, 1341, 1291, 1208, 1184, 1031, 954, 707, 391, 333, and 108. The bulk density of the darunavir dimethylsulfoxide solvate of the present invention is 0.472±0.008 g/ml, approximately 26 % denser when compared to the bulk density (0.374 ± 0.009 g/mL) of the known ethanolate form.

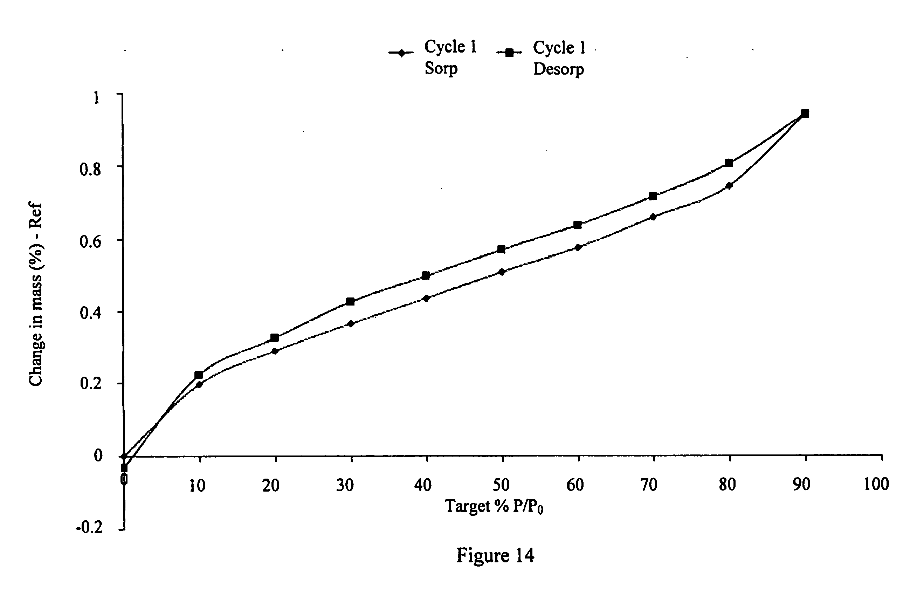

About 10mg of the dimethylsulfoxide solvate form of the present invention were used to test the hygroscopicity (DVS) from 0% to 90% according to the details on Table 2 hereinabove. The dimethylsulfoxide solvate form of the present invention was found to be only slightly hygroscopic (0.9431% weight gain from 0% to 90%; Figure 14).

The dimethylsulfoxide solvate of darunavir of the present invention was further evaluated for its chemical stability. The results are summarized in Table 6. Specifically, about 3 mg of the compound was accurately weighed into a 20 ml clear glass vial and stored under the following conditions: 40°C, 60°C, 40°C/RH 75%, 60°C/RH 75%, and light (25°C), for 2 weeks. A sample stored at -20°C was used as control. No significant drop in recovery values or increase in TRS was observed at 40 °C, 40 °C/75%RH and under exposure to light at 25 °C, while a slight increase was found at 60 °C and 60 °C/75%RH. The extent of degradation of the dimethylsulfoxide solvate of darunavir was less than 50% in comparison to the known ethanolate form, under the accelerated test conditions (60 °C and 60 °C/75%RH). Additionally, no change was observed in the physical appearance at the end of the 1^{st} and 2^{nd} week for samples stored at 40°C, 40°C/RH 75% and 25 °C under light.

**Table 6: Solid stability of darunavir dimethylsulfoxide solvate at 40°C, 60°C, 40°C/75%RH, 60°C/75%RH and under light exposure for 7 days and 14 days**

| Condition | Time | Sample number | Weight (mg) | Appearance | TRS% | Remaining% |
|---|---|---|---|---|---|---|
| -20°C | 7d | 1 | 2.881 | No change | 1.22 | - |
| | | 2 | 2.684 | No change | 1.22 | |
| | 14d | 1 | 1.932 | No change | 1.22 | - |
| | | 2 | 3.244 | No change | 1.27 | |
| 40°C | 7d | 1 | 2.226 | No change | 1.21 | 99.21 |
| | | 2 | 2.853 | No change | 1.21 | |
| | 14d | 1 | 3.557 | No change | 1.27 | 100.19 |
| | | 2 | 2.825 | No change | 1.27 | |
| 60°C | 7d | 1 | 2.957 | Stuck | 1.43 | 99.95 |
| | | 2 | 2.767 | Stuck | 1.29 | |
| | 14d | 1 | 2.755 | Stuck | 2.07 | 99.91 |
| | | 2 | 3.175 | Stuck | 2.12 | |
| 40°C/75%RH | 7d | 1 | 2.534 | No change | 1.22 | 99.54 |
| | | 2 | 2.786 | No change | 1.24 | |
| | 14d | 1 | 2.639 | No change | 1.23 | 100.31 |
| | | 2 | 2.640 | No change | 1.27 | |
| 50°C/75%RH | 7d | 1 | 2.129 | Stuck | 2.09 | 99.01 |
| | | 2 | 2.791 | Stuck | 2.08 | |
| | 14d | 1 | 3.352 | Stuck | 2.04 | 99.85 |
| | | 2 | 2.341 | Stuck | 1.91 | |
| light | 7d | 1 | 2.228 | No change | 1.29 | 99.59 |
| | | 2 | 2.615 | No change | 1.19 | |
| | 14d | 1 | 3.633 | No change | 1.22 | 100.12 |
| | | 2 | 2.724 | No change | 1.21 | |

The aqueous solubility of the dimethylsulfoxide solvate of darunavir of the present invention was measured. Specifically, about 10 mg of the compound was accurately weighed into a 4 ml clear glass vial followed by the addition of 2.5 ml buffer (at different pH) to the vial. The vial was then mounted on a Thermomixer and kept shaking for 24 hours at 25°C. The solution was filtered through 0.45 µm PTFE filter. The pH value and concentration of the filtrate were checked by pH meter and HPLC, respectively. The results are summarized in Table 7 and Figure 8 panel A.

**Table 7: Solubility results of darunavir dimethylsulfoxide solvate in aqueous buffers**

| Testing media | Solubility (mg/ml) | Appearance | Final pH |
|---|---|---|---|
| water | 0.23 | Many particles | 6.001 |
| pH 1.2 | 1.36* | Many particles | 1.236 |
| pH 4.5 | 0.22 | Many particles | 4.486 |
| pH 6.8 | 0.21 | Many particles | 6.785 |
| pH 7.4 | 0.20 | Many particles | 7.437 |

| | | | |
|---|---|---|---|
| *degraded | | | |

### Reference Example 5: Preparation of amorphous darunavir

The amorphous darunavir form was prepared by slow precipitation from a saturated solution using the following solvent systems: methyl isobutyl ketone (MIBK), isopropyl acetate (iPrOAc), acetonitrile (ACN), dichloromethane (DCM), ethyl acetate (EtOAc) wet and anhydrous, and in the following mixtures of solvents: ACN:Acetone (1:1), at 60°C with ACN: Toluene (1:6), DCM: MeOH (1:6), Acetone:MeOH (1:6), ACN:MTBE (1:9), Acetone:MTBE (1:9), 2-MeTHF:MeOH (1:8), THF:MeOH (1:9).

Alternatively, the amorphous form was prepared in the following solvent / antisolvent systems: methyl ethyl ketone (MEK) / Methyl tert-butyl ether (MTBE), CH₂Cl₂ / Toluene, acetonitrile (ACN) / H₂O, 2-MeTHF / IPA and MIBK / Toluene.

Alternatively, the amorphous form was prepared by dissolving about 1g of Darunavir ethanolate in 1.5ml CH₂Cl₂. CH₂Cl₂ was then evaporated under ambient conditions until a precipitate formed.

### Reference Example 6: Characterization of amorphous darunavir

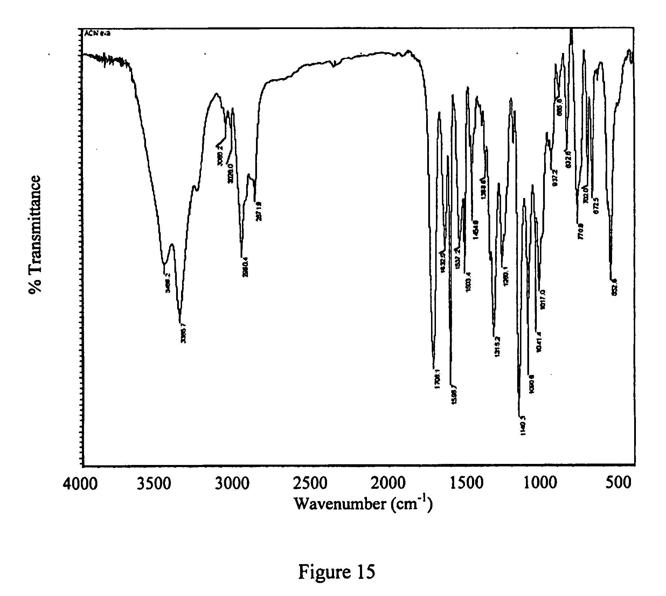

The amorphous darunavir showed a broad X-ray diffraction peak between 10 and 25 [2θ°] characteristic of an amorphous powder (Figure 10, panels A and E). The XRPD remained unchanged even after storage at 25°C for 2 weeks indicating stability of the amorphous form. The IR spectrum of the amorphous form is shown in Figure 15. Unique and specific spectral differences between the amorphous form of US 2005/0250845 and the amorphous form of the present invention appear in 2 spectral regions: 1500-1320cm⁻¹ (hereinafter region 1) and 800-500 cm⁻¹ (hereinafter region 2).

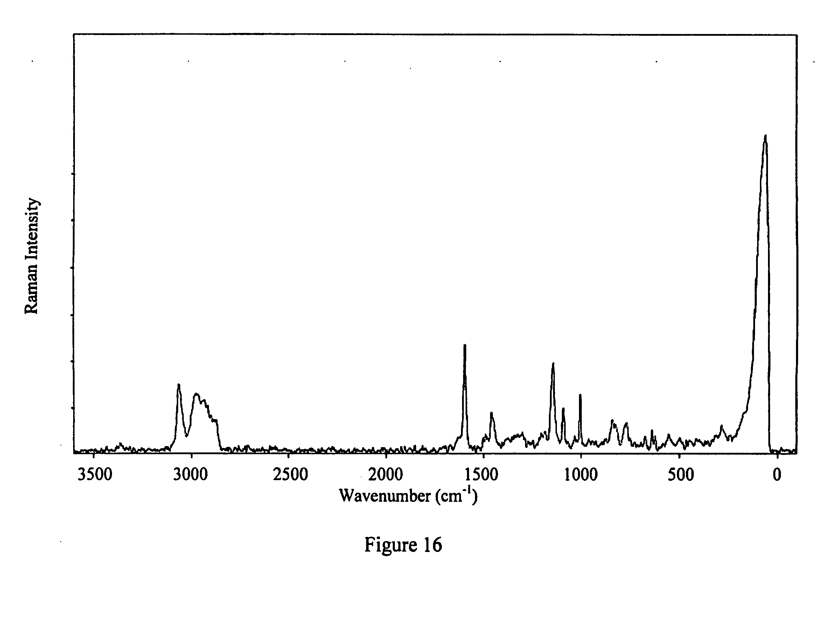

Specifically, whereas the amorphous form of US 2005/0250845 shows no peaks in region 1, the present amorphous form is characterized by two single absorption bands at 1454 and 1369 cm⁻¹. Additionally, the amorphous form of US 2005/0250845 shows 3 absorption bands at 750, 702 and 672 cm⁻¹ in region 2. The present amorphous form shows 2 additional peaks in this region, at 771 and 553 cm⁻¹. The Raman spectrum is shown in Figure 16. The characteristic Raman peaks of the present amorphous darunavir appear at about 61, 285, 553, 622, 673, 767, 841, 1004, 1091, 1145, 1459, 1597, 2931, 2966, and 3063 cm⁻¹. Differences in the Raman intensity between the present amorphous form and the known ethanolate form of darunavir appear at least at the following wavenumbers (cm⁻¹): 841, 622 and 61. The bulk density of the present amorphous darunavir is 0.445±0.012 g/ml.

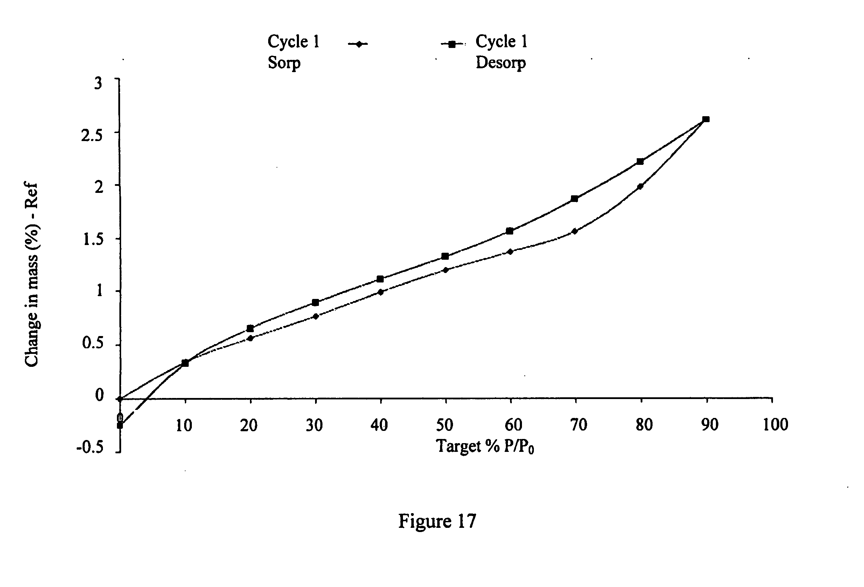

About 10mg of the present amorphous darunavir were used to test the hygroscopicity (DVS) from 0% to 90% according to the details on Table 2 hereinabove. The present amorphous form was found to be hygroscopic (2.617% weight gain from 0% to 90%); Figure 17).

The present amorphous darunavir form was further evaluated for its chemical stability. The results are summarized in Table 8. Specifically, about 3 mg of the compound was accurately weighed into a 20 ml clear glass vial and stored under the following conditions: 40°C, 60°C, 40°C/RH 75%, 60°C/RH 75%, and light (25°C), for 2 weeks. A sample stored at -20°C was used as control. A slight increase in TRS was found at 40 °C, 40 °C/75%RH, 60 °C and 60 °C/75%RH, while no increase was observed for the amorphous form that was stored under exposure to light at 25 °C, both at the end of 1^{st} and 2^{nd} weeks. Additionally, no change was observed in the physical appearance at the end of the 1^{st} and 2^{nd} week for samples stored at 40°C, 40°C/RH 75% and 25 °C under light.

**Table 8: Solid stability of amorphous darunavir at 40°C, 60°C, 40°C/75%RH, 60°C/75%RH and under light exposure for 7 days and 14 days**

| Condition | Time | Sample number | Weight (mg) | Appearance | TRS% | Remaining% |
|---|---|---|---|---|---|---|
| -20°C | 7d | 1 | 2.496 | No change | 2.05 | - |
| | | 2 | 2.210 | No change | 2.05 | |
| | 14d | 1 | 2.812 | No change | 2.07 | - |
| | | 2 | 3.434 | No change | 2.08 | |
| 40°C | 7d | 1 | 3.025 | No change | 3.03 | 97.22 |
| | | 2 | 2.861 | No change | 3.07 | |
| | 14d | 1 | 2.780 | No change | 3.35 | 97.40 |
| | | 2 | 2.991 | No change | 3.36 | |
| 60°C | 7d | 1 | 2.775 | Stuck | 3.97 | 96.60 |
| | | 2 | 2.780 | Stuck | 3.95 | |
| | 14d | 1 | 2.884 | Stuck | 4.04 | 96.08 |
| | | 2 | 2.575 | Stuck | 4.00 | |
| 40°C/75%RH | 7d | 1 | 2.726 | No change | 3.12 | 96.36 |
| | | 2 | 2.681 | No change | 3.29 | |
| | 14d | 1 | 2.385 | No change | 3.64 | 97.10 |
| | | 2 | 2.887 | No change | 3.78 | |
| 60°C/75%RH | 7d | 1 | 2.644 | Stuck | 4.17 | 94.64 |
| | | 2 | 2.660 | Stuck | 4.62 | |
| | 14d | 1 | 3.272 | Stuck | 3.97 | 96.62 |
| | | 2 | 2.765 | Stuck | 4.01 | |
| light | 7d | 1 | 2.575 | No change | 2.04 | 99.11 |
| | | 2 | 2.797 | No change | 2.07 | |
| | 14d | 1 | 2.924 | No change | 2.06 | 100.88 |
| | | 2 | 2.580 | No change | 2.06 | |

The aqueous solubility of the present amorphous darunavir was measured. Specifically, about 10 mg of the compound was accurately weighed into a 4 ml clear glass vial followed by the addition of 2.5 ml buffer (at different pH) to the vial. The vial was then mounted on a Thermomixer and kept shaking for 24 hours at 25°C. The solution was filtered through 0.45 µm PTFE filter. The pH value and concentration of the filtrate were checked by pH meter and HPLC, respectively. The results are summarized in Table 9 and in Figure 8 panel C.

**Table 9: Solubility results of amorphous darunavir in aqueous buffers**

| Testing media | Solubility (mg/ml) | Appearance | Final pH |
|---|---|---|---|
| water | 0.27 | Many particles | 5.765 |
| pH 1.2 | 1.73* | Many particles | 1.226 |
| pH 4.5 | 0.27 | Many particles | 4.534 |
| pH 6.8 | 0.24 | Many particles | 6.790 |
| pH 7.4 | 0.23 | Many particles | 0.428 |

| | | | |
|---|---|---|---|
| *degraded | | | |

### Example 7: Hygroscopicity

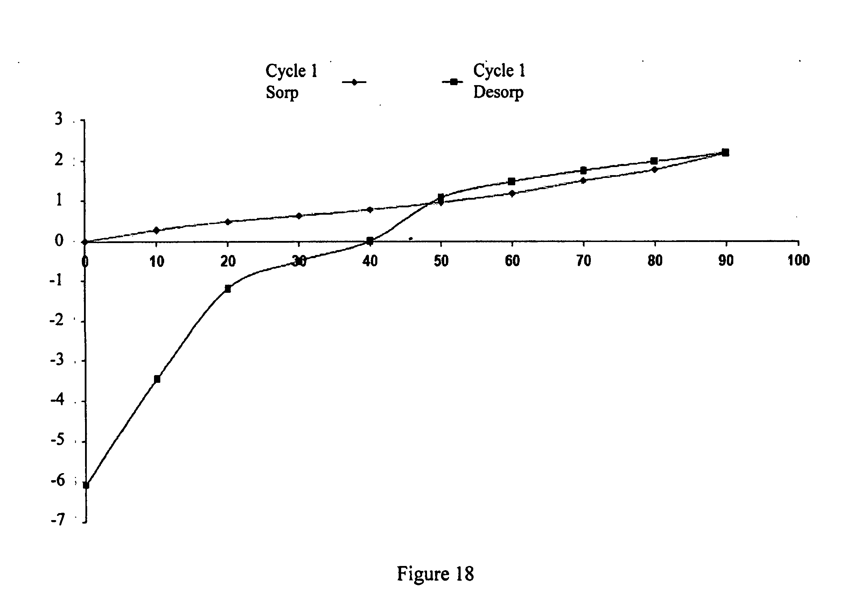

The hygroscopicity of the Darunavir form of the present invention was compared to the hygroscopicities of Darunavir ethanolate API and of darunavir THF. Whereas the tetrahydrofuran and dimethylsulfoxide solvates of the present invention were only slightly hygroscopic (1.366% and 0.9431% weight gain from 0% to 90%, respectively), darunavir ethanolate (API) was hygroscopic with 2.180% weight gain from 0% to 90% (Figure 18). Additionally, darunavir ethanolate lost the solvate ethanol molecules during the desorption measurement.

Thus, the tetrahydrofuran solvate and the dimethylsulfoxide solvate of the present invention may possess longer term stability in humid environments and are thus more advantageous for use in the pharmaceutical industry in comparison to darunavir ethanolate.

While the present invention has been particularly described, persons skilled in the art will appreciate that many variations and modifications can be made. Therefore, the invention is not to be construed as restricted to the particularly described embodiments, and the scope and concept of the invention will be more readily understood by reference to the claims, follow.

## Claims

1. A crystalline dimethylsulfoxide solvate of darunavir having an X-ray powder diffraction pattern with diffraction peaks at 2-theta values of about 20.6±0.1 and 21.2±0.1.

2. The crystalline dimethylsulfoxide solvate of darunavir according to claim 1
having an X-ray powder diffraction pattern with diffraction peaks at 2-theta values of about 20.6±0.1, 21.2±0.1, 16.6±0.1 and 23.0±0.1; or
having at least 3 X-ray diffraction peaks selected from about 7.1±0.1, 9.3±0.1, 10.6±0.1, 11.4±0.1, 13.9±0.1, 16.6±0.1, 17.3±0.1, 18.5±0.1, 20.1±0.1, 20.6±0.1, 21.2±0.1, 23.0±0.1, 27.1±0.1 and 28.1±0.1 degrees 2-theta; or
having an X-ray powder diffraction pattern with diffraction peaks at 2-theta values of about 7.1±0.1, 9.3±0.1, 10.6±0.1, 11.4±0.1, 13.9±0.1, 16.6±0.1, 17.3±0.1, 18.5±0.1, 20.1±0.1, 20.6±0.1, 21.2±0.1, 23.0±0.1, 27.1±0.1 and 28.1±0.1.

3. A pharmaceutical composition comprising as an active ingredient the crystalline dimethylsulfoxide solvate of darunavir according to any one of claims 1-2 and a pharmaceutically acceptable carrier.

4. The pharmaceutical composition according to claim 3 in the form of a tablet.

5. Crystalline dimethylsulfoxide solvate of darunavir according to any one of claims 1-2 for use as a medicament.

6. Crystalline dimethylsulfoxide solvate of darunavir according to any one of claims 1-2 for use in the treatment of retroviral infections.

7. Crystalline dimethylsulfoxide solvate of darunavir for use according to claim 6, wherein the retroviral infection is a human immunodeficiency virus (HIV) infection.

8. Crystalline dimethylsulfoxide solvate of darunavir for use according to claim 7 coadministered in combination with at least one other antiretroviral drug, preferably wherein the at least one other antiretroviral drug is ritonavir.

## Patentansprüche

1. Kristallines Dimethylsulfoxid-Solvat von Darunavir, welches ein Röntgen-Pulverdiagramm mit Streuungs-Peaks bei 2-theta-Werten von etwa 20,6±0,1 und 21,2±0,1 aufweist

2. Kristallines Dimethylsulfoxid-Solvat von Darunavir nach Anspruch 1,
welches ein Röntgen-Pulverdiagramm mit Streuungs-Peaks bei 2-theta-Werten von etwa 20,6±0,1 und 21,2±0,1, 16,6±0,1 und 23,0±0,1 aufweist; oder
mit mindestens 3 Röntgen-Streuungs-Peaks ausgewählt unter etwa 7,1±0,1, 9,3±0,1, 10,6±0,1, 11,4±0,1, 13,9±0,1, 16,6±0,1, 17,3±0,1, 18,5±0,1, 20,1±0,1, 20,6±0,1, 21,2±0,1, 23,0±0,1, 27,1±0,1 und 28,1±0,1 Grad 2-theta; oder
mit einem Röntgen-Pulverdiagramm mit Streuungs-Peaks bei 2-theta Werten von etwa 7,1±0,1, 9,3±0,1, 10,6±0,1, 11,4±0,1, 13,9±0,1, 16,6±0,1, 17,3±0,1, 18,5±0,1, 20,1±0,1, 20,6±0,1, 21,2±0,1, 23,0±0,1, 27,1±0,1 und 28,1±0,1.

3. Pharmazeutischen Zusammensetzung, welche als aktiven Inhaltsstoff das kristalline Dimethylsulfoxid-Solvat von Darunavir nach einem der Ansprüche 1-2 und einen pharmazeutisch annehmbaren Träger umfasst.

4. Pharmazeutische Zusammensetzung nach Anspruch 3 in Form einer Tablette.

5. Kristallines Dimethylsulfoxid-Solvat von Darunavir nach einem der Ansprüche 1 - 2 zur Verwendung als Medikament.

6. Kristallines Dimethylsulfoxid-Solvat von Darunavir nach einem der Ansprüche 1 - 2 zur Verwendung bei der Behandlung retroviraler Infektionen.

7. Kristallines Dimethylsulfoxid-Solvat von Darunavir zur Verwendung nach Anspruch 6, worin die retrovirale Infektion eine humane Immunschwäche (HIV-) Infektion ist.

8. Kristallines Dimethylsulfoxid-Solvat von Darunavir nach Anspruch 7, zusammen verabreicht in Kombination mit mindestens einem anderen anti-retroviralen Arzneimittel, vorzugsweise wobei das mindestens eine andere anti-retrovirale Arzneimittel Ritonavir ist.

## Revendications

1. Solvate cristallin de darunavir de type diméthylsulfoxyde présentant un diagramme de diffraction de rayons X sur poudre comportant des pics de diffraction à des valeurs 2 thêta d'environ 20,6±0,1 et 21,2±0,1.

2. Solvate cristallin de darunavir de type diméthylsulfoxyde selon la revendication 1
présentant un diagramme de diffraction de rayons X sur poudre comportant des pics de diffraction à des valeurs 2 thêta d'environ 20,6±0,1, 21,2±0,1, 16,6±0,1 et 23,0±0,1 ; ou
présentant au moins 3 pics de diffraction de rayons X choisis parmi ceux d'environ 7,1±0,1, 9,3±0,1, 10,6±0,1, 11,4±0,1, 13,9±0,1, 16,6±0,1, 17,3±0,1, 18,5±0,1, 20,1±0,1, 20,6±0,1, 21,2±0,1, 23,0±0,1, 27,1±0,1 et 28,1±0,1 degrés 2 thêta ; ou
présentant un diagramme de diffraction de rayons X sur poudre comportant des pics de diffraction à des valeurs 2 thêta d'environ 7,1±0,1, 9,3±0,1, 10,6±0,1, 11,4±0,1, 13,9±0,1, 16,6±0,1, 17,3±0,1, 18,5±0,1, 20,1±0,1, 20,6±0,1, 21,2±0,1, 23,0±0,1, 27,1±0,1 et 28,1±0,1.

3. Composition pharmaceutique comprenant comme principe actif le solvate cristallin de darunavir de type diméthylsulfoxyde selon l'une quelconque des revendications 1-2 et un support pharmaceutiquement acceptable.

4. Composition pharmaceutique selon la revendication 3, sous forme d'un comprimé.

5. Solvate cristallin de darunavir de type diméthylsulfoxyde selon l'une quelconque des revendications 1-2, destiné à une utilisation comme médicament.

6. Solvate cristallin de darunavir de type diméthylsulfoxyde selon l'une quelconque des revendications 1-2, destiné à une utilisation dans le traitement d'infections rétrovirales.

7. Solvate cristallin de darunavir de type diméthylsulfoxyde, destiné à une utilisation selon la revendication 6, l'infection rétrovirale étant une infection due au virus de l'immunodéficience humaine (VIH).

8. Solvate cristallin de darunavir de type diméthylsulfoxyde, destiné à une utilisation selon la revendication 7, coadministré en combinaison avec au moins un autre médicament antirétroviral, ledit au moins un autre médicament antirétroviral étant de préférence le ritonavir.
